(19) Europäisches Patentamt
European Patent Office
Office européen des brevets

(11) Veröffentlichungsnummer: **0 234 377**
**A1**

(12) # EUROPÄISCHE PATENTANMELDUNG

(21) Anmeldenummer: **87101699.4**

(22) Anmeldetag: **07.02.87**

(51) Int.Cl.³: **C 07 C 149/18**
C 10 M 135/24
//C10N30/06

(30) Priorität: **15.02.86 DE 3604793**

(43) Veröffentlichungstag der Anmeldung:
**02.09.87 Patentblatt 87/36**

(84) Benannte Vertragsstaaten:
**AT BE CH DE ES FR GB IT LI LU NL SE**

(71) Anmelder: **Henkel Kommanditgesellschaft auf Aktien**
**Postfach 1100 Henkelstrasse 67**
**D-4000 Düsseldorf-Holthausen(DE)**

(72) Erfinder: **Borggrefe, Gerhard, Dr.**
**Weselerstrasse9**
**D-4000 Düsseldorf(DE)**

(54) **Alkylsubstituierte 1,6-Dihydroxy-3,4-sulfahexane, Verfahren zu ihrer Herstellung und ihre Verwendung.**

(57) Die Erfindung betrifft Verbindungen der allgemeinen Formel I

$$\begin{array}{c}R^1\text{–}\underset{\displaystyle OH}{\underset{|}{CH}}\text{–}\underset{\displaystyle R^2}{\underset{|}{CH}}\text{–S–S–}\underset{\displaystyle R^2}{\underset{|}{CH}}\text{–}\underset{\displaystyle OH}{\underset{|}{CH}}\text{–}R^1\end{array} \qquad (I)$$

in der $R^1$ und $R^2$ gleich oder verschieden sein können und für Wasserstoff oder geradkettige Alkylreste mit 1 bis 18 C-Atomen stehen, wobei die Gesamtzahl der C-Atome der Reste $R^1$ und $R^2$ im Bereich von 4 bis 18 liegt, ein Verfahren zur Herstellung derartiger Verbindungen durch Umsetzung von Epoxiden der allgemeinen Formel II

$$\overset{O}{R^1\text{–CH–CH–}R^2} \qquad (II)$$

in der $R^1$ und $R^2$ die oben angegebenen Bedeutungen haben, mit einem Alkalimetalldisulfid im Molverhältnis 2 : 1 sowie die Verwendung der dabei erhaltenen Verbindungen der allgemeinen Formel (I) als Freß- und Verschleißschutz-Additive für Öle und Fette.

EP 0 234 377 A1

Patentanmeldung D7362 EP

0234377

HENKEL KGaA
ZR-FE/Patente
Dr.Zt/KK

13.Febr.1986

# Alkylsubstituierte 1.6-Dihydroxy-3.4-sulfahexane, Verfahren zu ihrer Herstellung und ihre Verwendung

Die Erfindung betrifft neue alkylsubstituierte 1.6-Dihydroxy-3.4-sulfahexane, ein Verfahren zur Herstellung dieser neuen Verbindungen und ihre Verwendung als Freß- und Verschleißschutzadditive.

Kohlenwasserstofföle natürlicher, halbsynthetischer oder vollsynthetischer Herkunft können ohne den Zusatz weiterer Stoffe nicht mehr alle Anforderungen erfüllen, die die Schmiertechnik in modernen Maschinen an sie stellt. Sie werden daher mit Zusatzstoffen, sogenannten Additiven, versetzt, mit denen sich ihre Eigenschaften gezielt und auf den jeweiligen Anwendungszweck abgestellt verbessern lassen. Insbesondere eine Minderung des Verschleißes mechanisch aneinanderreibender Metallteile sowie eine Erhöhung des Lastaufnahmevermögens zur Übertragung größerer Kräfte sind Ziele, die bei der Entwicklung moderner Schmiermittel-Additive verfolgt werden.

Zur Verschleißminderung und zur Erhöhung des Lasttragevermögens werden Motorenölen, Getriebeölen, Hydraulikölen und Turbinenölen sowie Metallbearbeitungsölen und deren Emulsionen sogenannte EP-(extreme pressure)- und AW-(anti wear)- Additive zugesetzt. Es ist bekannt, daß schwefelhaltige Verbindungen, in denen Schwefel in "disponibler" Form vorliegt, besonders gute Wirksamkeit als Additive für derartige Zwecke zeigen. Besonders wirksam sind Verbindungen mit Polysulfid-Brücken, in denen sich die Schwefelatome in einer labilen, aktiven Form befinden und bei höheren Temperaturen freigesetzt bzw. zur Bildung von Metallsulfid-Schichten auf der Metalloberfläche herangezogen werden können, die meist einen geringeren Reibungskoeffizient haben als die Metalle selbst und dadurch zu einer Verbesserung des Reibungsverhaltens beitragen (vgl. Ullmanns Enzyklopädie der technischen Chemie, Band 20, Seite 552 ff (1981)).

Verbindungen dieser Art werden meistens durch unmittelbare Schwefelung von Ölen, von einfach oder mehrfach ungesättigten Fettsäuren oder ihren Alkylestern, oder von Olefinen hergestellt. Häufig werden als Additive auch Mischungen derartiger, relativ hohe Schwefelanteile enthaltender Produkte verwendet. Außerdem haben als Verschleißschutz-Additive für Schmiermittel noch Metallsalze von Dialkyl- bzw. Diaryl-Dithiophosphaten, beispielsweise Zink-Dialkyldithiophosphate, oder Ester bzw. Salze der Carbaminsäure, z.B. Oleylcarbamat, große Bedeutung (vgl. Ullmanns Enzyklopädie, a.a.O.).

Aufgabe der vorliegenden Erfindung war es, neue Verbindungen zur Verfügung zu stellen, die in gleicher Weise wie bisher bekannte Verbindungen bei der Legierung von Mineralölen oder synthetischen Esterölen als EP- und AW-Additive geeignet sind und sich durch chemisch wenig aufwendige Verfahren aus preiswert zugänglichen Ausgangsstoffen herstellen lassen. Die aufzufindenden Verbindungen sollten dabei eine mit schon bekannten Additiven vergleichbare Wirkung zeigen und nicht in irgendeiner Weise toxisch bzw. umweltschädlich sein.

Es wurde nun überraschend gefunden, daß als die obigen Anforderungen erfüllende Additive alkylsubstituierte 1.6-Dihydroxy-3.4-sulfahexane hervorragend geeignet sind.

Die Erfindung betrifft alkylsubstituierte 1.6-Dihydroxy-3.4-sulfahexane der allgemeinen Formel (I)

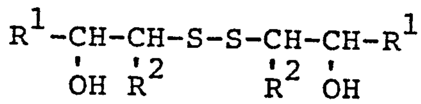

(I)

in der $R^1$ und $R^2$ gleich oder verschieden sein können und für Wasserstoff oder geradkettige Alkylreste mit 1 bis 18 C-Atomen stehen, wobei die Gesamtzahl der C-Atome der Reste $R^1$ und $R^2$ im Bereich von 4 bis 18 liegt.

Die Erfindung betrifft außerdem ein Verfahren zur Herstellung alkylsubstituierter 1.6-Dihydroxy-3.4-sulfahexane der allgemeinen Formel (I) mit den obigen Bedeutungen für $R^1$ und $R^2$, das dadurch gekennzeichnet ist, daß man durch Epoxidation von Monoolefinen erhaltene Epoxide der allgemeinen Formel (II)

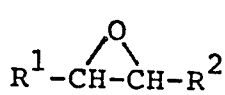

(II)

in der $R^1$ und $R^2$ die oben angegebenen Bedeutungen haben, in einem polaren Lösungsmittel oder Lösungsmittelgemisch bei Temperaturen im Bereich von 0 bis 100°C mit einem Alkalisulfid der allgemeinen Formel (III)

$$M_A-S-(S)_n-S-M_A \qquad (III)$$

in der $M_A$ für ein Alkalimetall steht und n 0 oder 2 bedeutet, im Molverhältnis 2 : 1 umsetzt und das Produkt nach an sich bekannten Methoden isoliert und reinigt.

Die Erfindung betrifft auch die Verwendung der alkylsubstituierten 1.6-Dihydroxy-3.4-sulfahexane der oben angegebenen allgemeinen Formel (I) in Mengen von 0,5 bis 20 Gew.-%, bevorzugt 1 bis 10 Gew.-%, bezogen auf die Ölbasis, als Freß- und Verschleißschutz-Additive für Schmieröle, Schmierfette, Kraftübertragungsöle und Metallbearbeitungsemlusionen aus Mineralöl- oder Syntheseölbasis.

Die erfindungsgemäßen Verbindungen der allgemeinen Formel (I) sind neue Verbindungen, die im Stand der Technik nicht beschrieben sind. In der allgemeinen Formel (I) können die organischen Reste $R^1$ und $R^2$ gleich oder verschieden sein. Sie können dabei für Wasserstoff oder geradkettige Alkylreste mit 1 bis 18 C-Atomen stehen, wobei die Gesamtzahl der C-Atome beider Reste $R^1$ und $R^2$ im Bereich von 4 bis 18 liegt.

Als Alkylreste kommen somit die n-Alkylreste aus der Gruppe Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl

und Octadecyl in Frage. Daß die Gesamtzahl der C-Atome beider Reste im Bereich von 4 bis 18 liegen muß bedeutet, daß sich die Zahl der C-Atome beider Reste mindestens auf die Zahl 4 und höchstens auf die Zahl 18 addieren muß. Steht beispielsweise einer der beiden Reste für Methyl, so kann der andere Rest aus der oben genannten Gruppe der Alkylreste aus der Gruppe von Propyl bis Heptadecyl ausgewählt werden. Steht allerdings einer der beiden Reste für Wasserstoff, so kommen für den anderen Rest nur Alkylreste aus dem Bereich von Butyl bis Octadecyl in Frage.

Bevorzugt werden Verbindungen der allgemeinen Formel (I), in denen $R^1$, also der endständige Alkylrest, für eine geradkettige Alkylgruppe mit 6 bis 14 C-Atomen und $R^2$, also der Alkylrest in 2-Position, für Wasserstoff steht. Besonders haben sich diejenigen Verbindungen der allgemeinen Formel (I) bewährt, in denen $R^1$ für geradzahlige Alkylreste mit 6, 10 oder 14 C-Atomen und $R^2$ für Wasserstoff steht.

Das erfindungsgemäße Verfahren zur Herstellung der neuen Verbindungen der allgemeinen Formel (I) folgt dem nachfolgenden Reaktionsschema.

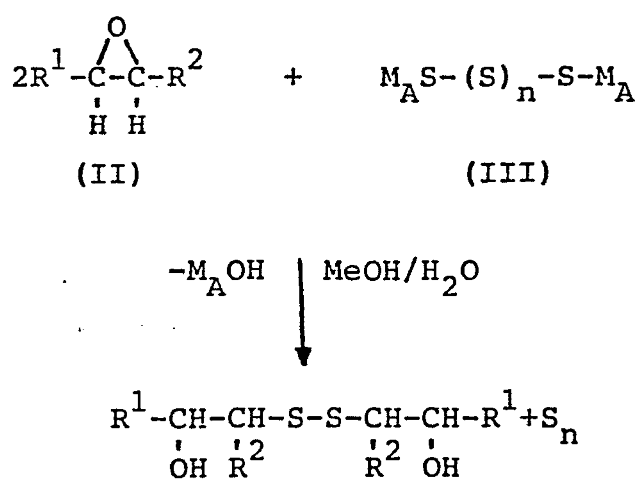


$n = 0,2$

$M_A$ = Alkalimetall

(I)

Die Verbindungen der allgemeinen Formel (I) lassen sich also dadurch herstellen, daß man Epoxide der allgemeinen Formel (II), in denen $R^1$ und $R^2$ gleich oder verschieden sein können und für Wasserstoff oder geradkettige Alkylreste mit 1 bis 18 C-Atomen stehen, mit Alkalisulfiden der allgemeinen Formel (III) im Molverhältnis 2 : 1 umsetzt.

Für die Reste $R^1$ und $R^2$ gilt auch in den zur Herstellung verwendeten Epoxiden die Beschränkung, daß die Gesamtzahl der C-Atome beider Reste $R^1$ und $R^2$ im Bereich von 4 bis 18 liegt. Dies bedeutet, daß als Epoxide geradkettige Verbindungen aus der Gruppe Epoxyhexen, Epoxyhepten, Epoxyocten, Epoxynonen, Epoxydecen, Epoxyundecen, Epoxydodecen, Epoxytridecen, Epoxytetradecen, Epoxypentadecen, Epoxyhexadecen, Epoxyheptadecen, Epoxyoctadecen, Epoxynonadecen und Epoxyeicosen in Frage kommen. Dabei ist es gleichgültig, in welcher Stellung des Epoxid-Moleküls der allgemeinen Formel (II) der Oxyranring steht: 1.2-Epoxyolefine sind gleichermaßen für das erfindungsgemäße Herstellungsverfahren geeignet wie Verbindungen, in denen der Oxyranring nicht endständig ist.

Die in dem erfindungsgemäßen Verfahren eingesetzten Epoxide der allgemeinen Formel (II) werden nach an sich bekannten Verfahren durch Epoxidation von entsprechenden Monoolefinen erhalten. So lassen sich beispielsweise 1.2-Monoolefine durch Epoxidation mit Peroxycarbonsäuren in Verbindungen der allgemeinen Formel (II) überführen, in denen $R^1$ für einen entsprechenden linearen Alkylrest und $R^2$ für Wasserstoff steht. Gleichermaßen sind jedoch auch Monoolefine mit innenständiger Doppelbindung auf demselben Wege in

Epoxide(II) zu überführen; in den resultierenden Verbindungen stehen dann die Reste $R^1$ und $R^2$ für Alkylreste der entsprechenden Kettenlänge. Bevorzugt werden solche Epoxide(II) für das erfindungsgemäße Herstellungsverfahren verwendet, die durch Epoxidation von 1.2-Monoolefinen mit 6 bis 20 C-Atomen gewonnen werden können.

Die oben beschriebenen Verbindungen der allgemeinen Formel (II) werden erfindungsgemäß mit einem Alkalidisulfid der oben angegebenen allgemeinen Formel (III) umgesetzt. In dieser Formel steht $M_A$ für ein Alkalimetall, beispielsweise Natrium oder Kalium. Erfindungsgemäß sind für die Umsetzung sowohl Alkalimetalldisulfide als auch Alkalimetalltetrasulfide verwendbar; entsprechend steht in der oben angegebenen allgemeinen Formel (III) n für 0 oder 2. Das Molverhältnis der Reaktionspartner Epoxid und Alkalimetallsulfid beträgt erfindungsgemäß 2 : 1. Wie sich aus dem obigen Reaktionsschema ergibt, wird bei der Verwendung von Alkalimetalltetrasulfid, das insbesondere in Form von Dinatriumtetrasulfid auch in großtechnischen Mengen preiswert verfügbar ist und daher bevorzugt verwendet wird, elementarer Schwefel $S_n$ abgespalten.

Die Umsetzung wird bei Temperaturen von 0 bis 100°C, bevorzugt bei Temperaturen im Bereich zwischen 20 und 70°C, in einem polaren Lösungsmittel oder einem Gemisch polarer Lösungsmittel durchgeführt. Als polares Lösungsmittel läßt sich beispielsweise Wasser verwenden. Bevorzugt werden jedoch Gemische polarer Lösungsmittel eingesetzt, die vorteilhafterweise aus Wasser und einem linearen aliphatischen Alkohol mit 1 bis 6 C-Atomen im Alkylrest bestehen. Bevorzugt werden

solche Lösungsmittelgemische eingesetzt, die aus Wasser und einem linearen aliphatischen Alkohol aus der Gruppe Methanol, Ethanol und Isopropanol oder auch einer Mischung aus Wasser und zweien oder allen dreien der genannten Alkohole bestehen.

Sofern erforderlich, kann die Reaktionsmischung zur Vervollständigung der Umsetzung noch eine gewisse Zeit, beispielsweise 30 bis 60 min, bei erhöhter Temperatur gehalten werden. Das Reaktionsprodukt der allgemeinen Formel (I) wird nach an sich bekannten Methoden isoliert und gereinigt. Dies erfolgt beispielsweise dadurch, daß die Polarität der Reaktionsmischung durch Zugabe von Wasser erhöht und das erfindungsgemäße Addukt in kristalliner Form ausgefällt wird. Die Kristalle werden durch Filtration aus der Reaktionsmischung abgetrennt und - sofern gewünscht - durch Umfällen oder Umkristallisieren bzw. Destillation, gereinigt.

Die auf dem beschriebenen Wege erhaltenen Verbindungen der allgemeinen Formel (I) lassen sich, wie überraschend gefunden wurde, mit zahlreichen natürlichen und/oder synthetischen Ölen legieren und erhöhen dabei wesentlich deren Lasttragevermögen und Verschleißschutz-Eigenschaften. Sie werden dazu in Mengen von 0,5 bis 20 Gew.-%, vorzugsweise in Mengen zwischen 1 bis 10 Gew.-%, bezogen auf die Ölbasis, verschiedenen Ölen für Zwecke der Schmierung oder Kraftübertragung, wie Mineralölen oder synthetischen Esterölen, ölhaltigen Emulsionen auf Mineralöl- oder Syntheseölbasis für die Metallbearbeitung oder auch Schmierfetten beigemischt. In den genannten Substanzen verbessern sie die Alterungsbeständigkeit und wirken auch bei erhöh-

..

ter Temperatur nicht korrosiv auf die mit den Ölen oder ölhaltigen Emulsionen in Kontakt kommenden Metalloberflächen, beispielsweise auf Buntmetalle.

Die Erfindung wird durch die nachfolgenden Beispiele näher erläutert.

Beispiel 1

Einer Lösung von 322 g (1 mol) 83 %igem 1.2-Epoxydodecen (Epoxidsauerstoff: 7,2 %) in 500 ml Methanol wurden 250 g (0,5 mol) einer 35 bis 38 %igen wässrigen Dinatriumtetrasulfidlösung portionsweise zugesetzt. Nach Abschluß der exothermen Reaktion wurde die Lösung noch eine Stunde bei 60°C gehalten und anschließend mit Wasser versetzt. Dabei fiel eine farblose kristalline Substanz aus, die durch Filtration abgetrennt und aus Methanol umgefällt wurde.

Ausbeute: 208 g (95 % der Theorie);
Schmelzpunkt: 52°C.

Analysenwerte

| | |
|---|---|
| S-Gehalt: | Berechnet: 14,75 % |
| | Gefunden: 14,5 %. |
| Molekulargewicht: | Berechnet: 434 |
| | (osmometrisch in Ethanol) |
| | Gefunden: 403. |

Beispiel 2

In gleicher Verfahrensweise wie in Beispiel 1 wurden 51,6 g (0,2 mol) 1.2-Epoxyhexadecen (Epoxidsauerstoff: 6,2 %) in Methanol mit 50 g (0,1 mol) $Na_2S_4$-Lösung in

Wasser umgesetzt. Nach Beendigung der exothermen Reaktion kristallisierte ein farbloses Festprodukt aus der Lösung aus, das durch Filtration abgetrennt wurde.

Ausbeute: 44 g (75 % der Theorie);
Schmelzpunkt: 72°C.

Analysenwerte
S-Gehalt: Berechnet: 11,7 %
Gefunden: 11,8 %.

Beispiel 3

In gleicher Verfahrensweise wie in Beispiel 1 wurden 200 g (1,56 mol) 1.2-Epoxyocten (Epoxidsauerstoff: 12,4 %) in Methanol mit 387 g (0,78 mol) $Na_2S_4$-Lösung in Wasser umgesetzt. Nach Beendigung der Reaktion wurde Wasser zugegeben. Dabei schied sich ein farbloses Öl ab, das separiert wurde (190 g, entsprechend 76 % der Theorie).

Ein Teil des Rohproduktes (150 g) wurde im Vakuum destilliert. Dabei wurden 33 g eines farblosen Öles mit einem Schwefelgehalt von 0,3 % gewonnen (Siedepunkt: 45°C/6,7 mbar). Als Rückstand verblieb ein dunkelbraunes Öl.

Ausbeute: 117 g.

Analysenwerte

| | | |
|---|---|---|
| S-Gehalt: | Berechnet: | 19,9 % |
| | Gefunden: | 19,4 %. |
| Molekulargewicht: | Berechnet: | 322 |
| | (osmometrisch in Ethanol) | |
| | Gefunden: | 300. |

Beispiel 4

Prüfung auf Eignung als Freß- und Verschleißschutz-Additive

Die erfindungsgemäßen Substanzen wurden in einem paraffinischen Mineralöl und einem synthetischen Esteröl im Shell-Vierkugelapparat nach DIN 51 350 auf ihre Eignung als Freß- und Verschleißschutz-Additive geprüft. Das Prüfverfahren wurde wie folgt durchgeführt:

In einem Stahlkugeltopf glitt in Anwesenheit des Schmierstoffes eine rotierende Laufkugel über drei ihr in Material und Durchmesser gleiche, fixierte Standkugeln. Der auf dem Vierkugelsystem lastende Druck konnte durch stufenweise Veränderung der Prüfkraft variiert werden.

Bei der Ermittlung der EP-Eigenschaften wurde in aufeinanderfolgenden Prüfläufen von je einer Minute Dauer die vorgegebene Prüfkraft so lange schrittweise gesteigert, bis ein Verschweißen des Vierkugelsystems eintrat.

Bei der Ermittlung der verschleißmindernden Eigenschaften wurde in Prüfläufen von je einer Stunde Dauer - bei Vorgabe unterschiedlicher Prüfkräfte - am Ende eines jeden Laufes der durch Verschleiß veränderte Kalottendurchmesser der drei Standkugeln gemessen und aus mehreren Messungen der Mittelwert bestimmt.

Die Ergebnisse sind der nachfolgenden Tabelle 1 zu entnehmen.

Tabelle 1

Prüfung der EP- und AW-Eigenschaften S-haltiger Epoxidfolgeprodukte nach DIN 51 350

| Additiv (I) $R^1$ | $R^2$ | S-Gehalt Gew-% | Öl | Konz. Gew.-% | Schweißkraft N | Kalottendurchmesser (mm) bei einem Prüfdruck von 300N | 450N | 600N |
|---|---|---|---|---|---|---|---|---|
| $C_{10}H_{21}$ | H | 14,5 | Mineralöl | 1 | - | 0,4 | 0,6 | 0,8 |
| | | | | 5 | 2400 | - | - | - |
| | | | Esteröl+) | 1 | - | 0,4 | 0,5 | 0,7 |
| | | | | 5 | 2400 | - | - | - |
| $C_{14}H_{29}$ | H | 11,8 | Mineralöl | 1 | - | 0,4 | 0,6 | 0,8 |
| | | | | 5 | 2200 | - | - | - |
| $C_6H_{13}$ | H | 16,9 | Mineralöl | 1 | - | 0,4 | 0,5 | 0,7 |
| | | | | 5 | 3000 | - | - | - |
| Geschwefelter Fettsäureester (Handelsprodukt) (Vgl.-Bsp. 1) | | 11 | Mineralöl | 1 | - | 0,4 | 0,5 | 0,9 |
| | | | | 5 | 2400 | - | - | - |
| Ohne Additiv | | 0 | Mineralöl | 0 | 1100 | 0,65 | 0,8 | 1,5 |
| (Vgl.-Bsp. 2) | | 0 | Esteröl+) | 0 | 1300 | 0,5 | 0,7 | 1,4 |

+) Trimethylolpropan-tri-$C_{8/10}$-fettsäureester

Vergleichsbeispiel 1

Entsprechend der in Beispiel 4 angegebenen Prüfmethode wurde ein kommerziell erhältliches Additiv (ein geschwefelter Fettsäureester) in Konzentrationen von 1 und 5 Gew.-% dem Mineralöl als Freß- und Verschleißschutz-Additiv zugesetzt. Die in Analogie zu Beispiel 4 erhaltenen Meßwerte sind ebenfalls der obigen Tabelle 1 zu entnehmen.

Vergleichsbeispiel 2

Entsprechend der in Beispiel 4 angegebenen Prüfmethode wurde ein natürliches Mineralöl und ein synthetisches Esteröl geprüft, das keine Additivzusätze enthielt. Die Ergebnisse sind ebenfalls der obigen Tabelle 1 zu entnehmen.

Ergebnis:
Wie sich aus einem Vergleich der Meßwerte ergibt, weisen die erfindungsgemäßen Verbindungen der allgemeinen Formel (I) mit kommerziell erhältlichen Verbindungen gleichwertige Eigenschaften als Freß- und Verschleißschutz-Additive auf und sind Ölen, die derartige Additive nicht enthalten, deutlich überlegen. Dies betrifft sowohl die Eigenschaften als EP-Additiv als auch die verschleißmindernden Eigenschaften.

Beispiel 5

Die erfindungsgemäßen Substanzen der allgemeinen Formel (I) wurden nach DIN 51 759 als Additive in Mineralöl hinsichtlich ihres korrosiven Charakters gegenüber Kupfermetall geprüft. Das Prüfverfahren wurde wie

folgt durchgeführt: Ein frisch geschmirgelter Kupferstreifen wurde bei 100°C über 3 h in eine 1 %ige Lösung des jeweiligen Additivs in Mineralöl getaucht. Nach 1 h wurde der Streifen der Lösung entnommen und durch Vergleich mit ASTM-Vergleichsstreifen der Korrosionsgrad ermittelt. Die Bewertung wurde wie folgt vorgenommen:

1: leichte Anlauffarben;
2: mäßige Anlauffarben;
3: starke Anlauffarben;
4: Korrosion.

Die Ergebnisse sind der nachfolgenden Tabelle 2 zu entnehmen.

Tabelle 2

Prüfung der korrosiven Eigenschaften S-haltiger Epoxidfolgeprodukte nach DIN 51 759
3 h/100°C, Cu-Streifen: entfettet, geschmirgelt, 1 %ig in Mineralöl

| Additiv (I) $R^1$ | $R^2$ | Korrosionsgrad |
|---|---|---|
| $C_{10}H_{21}$ | H | 1 |
| $C_{14}H_{29}$ | H | 1 |
| $C_6H_{13}$ | H | 1 |

Beispiel 6

Gemäß DIN 51 587 wurden die erfindungsgemäßen Verbindungen (I) in paraffinischen Mineralölen auf ihre Eignung als Radikalfänger bei der Stabilisierung des je-

weiligen Öls geprüft. Das Prüfverfahren wurde wie folgt durchgeführt:

300 ml Mineralöl, das die erfindungsgemäßen Substanzen als Additive enthielt, wurden in Anwesenheit von 60 ml Wasser und einer Doppeldrahtwendel, bestehend aus frisch geschmirgeltem Stahl- und Kupferdraht, bei 95°C und gleichzeitiger Begasung der Mischung mit 5 l Luft/h über 1000 h gealtert. Nach 100, 500 und 1000 h wurde jeweils die Veränderung der Neutralisationszahl des legierten Mineralöles bestimmt. Die Ergebnisse sind der nachfolgenden Tabelle 3 zu entnehmen.

Vergleichsbeispiel 3

Die Stabilitätsprüfung gemäß Beispiel 6 wurde an Mineralölen durchgeführt, die herkömmliche Handelsprodukte als Additive enthielten. Als solche wurden
(a) Zinkdialkyldithiophosphat und
(b) ein geschwefelter Fettsäureester
verwendet. Die Ergebnisse sind ebenfalls der nachfolgenden Tabelle 3 zu entnehmen.

Vergleichsbeispiel 4

Der Stabilistätsprüfung gemäß Beispiel 6 wurde auch ein Mineralöl, das kein Additiv enthielt, unterzogen. Die Ergebnisse der Prüfung sind ebenfalls der nachfolgenden Tabelle 3 zu entnehmen.

Tabelle 3

| Additiv (I) $R^1$ | $R^2$ | S-Gehalt (%) | Säurezahl nach Stunden 100 | 500 | 1000 |
|---|---|---|---|---|---|
| $C_{10}H_{21}$ | H | 14,5 | 0,25 | 0,35 | 0,41 |
| $C_6H_{13}$ | H | 16,9 | 0,2 | 0,4 | 0,8 |
| Zinkdialkyldithiophosphat (Vgl.-Bsp.3a) | | 16 | 0,1 | 0,2 | 0,67 |
| geschwefelter Fettsäureester (Vgl.-Bsp. 3b) | | 11 | 0,4 | 0,8 | 5,3 |
| ohne Additiv (Vgl.-Bsp. 4) | | 0 | 5,3 | 8,3 | 11,3 |

## Patentansprüche

1. Alkylsubstituierte 1.6-Dihydroxy-3.4-sulfahexane der allgemeinen Formel (I)

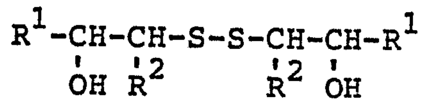

(I)

in der $R^1$ und $R^2$ gleich oder verschieden sein können und für Wasserstoff oder geradkettige Alkylreste mit 1 bis 18 C-Atomen stehen, wobei die Gesamtzahl der C-Atome der Reste $R^1$ und $R^2$ im Bereich von 4 bis 18 liegt.

2. Verbindungen der allgemeinen Formel (I) nach Anspruch 1, dadurch gekennzeichnet, daß $R^1$ für einen geradkettigen Alkylrest mit 6 bis 14 C-Atomen und $R^2$ für Wasserstoff steht.

3. Verfahren zur Herstellung alkylsubstituierter 1.6-Dihydroxy-3.4-sulfahexane der allgemeinen Formel (I)

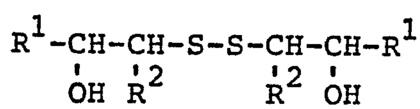

(I)

in der $R^1$ und $R^2$ gleich oder verschieden sein können und für Wasserstoff oder geradkettige Alkylreste mit 1 bis 18 C-Atomen stehen, wobei die Gesamtzahl der C-Atome der Reste $R^1$ und $R^2$ im Bereich von 4 bis 18 liegt, dadurch gekennzeichnet, daß man durch Epoxidation von Monoolefinen erhaltene Epoxide der allgemeinen Formel (II)

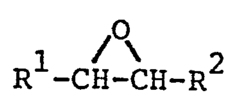

(II)

in der $R^1$ und $R^2$ die oben angegebenen Bedeutungen haben, in einem polaren Lösungsmittel oder polaren Lösungsmittelgemisch bei Temperaturen im Bereich von 0 bis 100°C mit einem Alkalimetallsulfid der allgemeinen Formel (III)

$$M_A-S-(S)_n-S-M_A \qquad (III)$$

in der $M_A$ für ein Alkalimetall steht und n 0 oder 2 bedeutet, im Molverhältnis 2 : 1 umsetzt und das Produkt nach an sich bekannten Methoden isoliert und reinigt.

4. Verfahren nach Anspruch 3, dadurch gekennzeichnet, daß man durch Epoxidation von 1.2-Monoolefinen mit 6 bis 20 C-Atomen gewonnene Epoxide verwendet.

5. Verfahren nach Ansprüchen 3 und 4, dadurch gekennzeichnet, daß man die Epoxide der allgemeinen Formel (II) mit Dinatriumdisulfid oder Dinatriumtetrasulfid umsetzt.

6. Verfahren nach Ansprüchen 3 bis 5, dadurch gekennzeichnet, daß man als polares Lösungsmittel Wasser verwendet.

7. Verfahren nach Ansprüchen 3 bis 5, dadurch gekennzeichnet, daß man als Lösungsmittelgemisch eine Mischung aus Wasser und einem oder mehreren linearen aliphatischen Alkoholen mit 1 bis 6 C-Atomen im Alkylrest verwendet.

8. Verfahren nach Anspruch 7, dadurch gekennzeichnet, daß man eine Mischung aus Wasser und einem oder mehreren Alkoholen aus der Gruppe Methanol, Ethanol und Isopropanol verwendet.

9. Verfahren nach Ansprüchen 3 bis 8, dadurch gekennzeichnet, daß man die Umsetzung im Temperaturbereich zwischen 20 und 70°C durchführt.

10. Verwendung der Verbindungen der allgemeinen Formel (I) nach Anspruch 1 in Mengen von 0,5 bis 20 Gew.-%, bevorzugt in Mengen von 1 bis 10 Gew.-%, bezogen auf die Ölbasis, als Freß- und Verschleißschutz-Additive für Schmieröle, Schmierfette, Kraftübertragungsöle und Metallbearbeitungsemulsionen auf Mineralöl- oder Syntheseölbasis.

Europäisches Patentamt

# EUROPÄISCHER RECHERCHENBERICHT

0234377

Nummer der Anmeldung

EP 87 10 1699

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| X | GB-A-2 111 053 (SANDOZ)<br>* Anspruch 13,vi *<br>--- | 1 | C 07 C 149/18<br>C 10 M 135/24 //<br>C 10 N 30/06 |
| X | CHEMICAL ABSTRACTS, Band 73, Nr. 5, 3. August 1970, Seiten 239-240, Zusammenfassung Nr. 28862p, Columbus, Ohio, US; H.G. NOESLER: "Inactivation of organotin compounds", & GESUNDHEITSW. DESINFEK. 1970, 62(1), 10-14<br>--- | 1,2 | |
| X | GB-A-1 052 214 (STAMICARBON)<br>* Seite 1 *<br>--- | 3-6 | |
| A | GB-A-2 085 918 (EXXON)<br>* Seite 2, Formel *<br>----- | | RECHERCHIERTE SACHGEBIETE (Int. Cl.4)<br>C 07 C 149/00 |

Der vorliegende Recherchenbericht wurde für alle Patentansprüche erstellt.

| Recherchenort | Abschlußdatum der Recherche | Prüfer |
|---|---|---|
| DEN HAAG | 12-05-1987 | VAN GEYT J.J.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82